# EUROPÄISCHE PATENTSCHRIFT

(11) **EP 2 074 957 B1**
(45) Veröffentlichungstag und Bekanntmachung des Hinweises auf die Patenterteilung: **17.04.2013**
(21) Anmeldenummer: 07150489.8
(22) Anmeldetag: 31.12.2007
(51) Int. Cl.: A61B 17/70

(54) **Pedikelschraube mit einer Verschlusseinrichtung zur Befestigung eines Stabes zur Stabilisierung der Wirbelsäule**
Pedicle screw with a locking device for attaching a rod to stabilise the spine
Vis pédiculaire dotée d'un dispositif de verrouillage destiné à la fixation d'une tige en vue de la stabilisation de la colonne vertébrale

(43) Veröffentlichungstag der Anmeldung: 01.07.2009
(73) Patentinhaber: Spinelab AG, 8406 Winterthur (CH)
(72) Erfinder: Zehnder, Thomas, 8806 Bäch (CH); Braunschweiler, Reto, 8413 Neftenbach (CH)
(74) Vertreter: BOVARD AG

(56) Entgegenhaltungen:
- EP-A- 1 234 547
- EP-A- 1 815 812
- WO-A-95/01132
- WO-A-2005/102195
- WO-A-2007/130840
- DE-U1- 9 403 231
- US-A1- 2003 187 434
- US-B2- 7 294 128

## Beschreibung

Die vorliegende Erfindung bezieht sich auf eine Pedikelschraube mit einer Verschlusseinrichtung zur Befestigung eines Stabes zur Stabilisierung der Wirbelsäule, umfassend einen Einschraubteil, einen am Einschraubteil angebrachten Kopfteil, eine im Kopfteil angeordnete -förmige Ausnehmung, die durch die inneren Oberflächen zweier Schenkel und einer die beiden inneren Oberflächen verbindenden Grundfläche gebildet ist, in welche Ausnehmung der Stab einsetzbar und durch die Verschlusseinrichtung gehalten ist, welche Verschlusseinrichtung ein bügelförmiges Verriegelungselement umfasst, welches an den beiden einander gegenüberliegenden Endbereichen jeweils einen Kragen aufweist, an welchen jeweils gegeneinander gerichtete Nocken angebracht sind, und aussenseitig an den beiden Schenkeln jeweils eine quer zu diesen verlaufende Nut angebracht ist, derart dass das bügelförmige Verriegelungselement auf die Schenkel aufsetzbar und verdrehbar ist und die Nocken zum Erreichen der Verriegelungsposition jeweils in die entsprechende Nut gelangen, und in das bügelförmigen Verriegelungselement eine Spannschraube eingeschraubt ist.

Pedikelschrauben mit einer Verschtusseinrichtung zur Befestigung eines Stabes sind in vielfältiger Weise aus dem Stand der Technik bekannt. Diese dienen zur Stabilisierung der Wirbelsäulen von Patienten, welche Wirbelsäulen starke Schäden aufweisen. Zur Stabilisierung wird in eine Anzahl von Wirbelkörpern jeweils eine Pedikelschraube eingeschraubt, in die Kopfteile dieser Pedikelschrauben wird eine Stange eingelegt, welche dann jeweils mit der entsprechenden Pedikelschraube verbunden wird, wozu Verschlusseinrichtungen verwendet werden. Je nachdem, welche Art von Stange man einsetzt, können zwei unterschiedliche Stabilisierungsformen erreicht werden. Beim Einsatz einer starren Stange erreicht man eine Versteifung der betroffenen Wirbelkörper, wenn eine stützende Stabilisierung der Wirbelkörper erreicht werden soll, wird eine elastische Stange eir gesetzt, wodurch erreicht wird , dass eine gewisse Beweglichkeit zwischen den einzelnen Wirbelkörpern zugelassen wird.

Unabhängig davon, welches System eingesetzt wird, ist eine optimale Verbindung zwischen eingesetzter Stange und Pedikelschraube anzustreben, was durch die eingesetzten Verschlusseinrichtungen erreicht werden soll.

Aus der EP-B-1 119 304 ist eine Vorrichtung zur Befestigung von Wirbelsäulenstangen bekannt, welche aus einer Pedikelschraube besteht, die einen Kopfteil mit einer U-förmigen Ausnehmung aufweist, in welche die zu fixierende Stange eingesetzt wird. Als Verschlusseinrichtung dient ein verdrehbares Element mit zwei einander gegenüberliegenden, seitlich vorstehenden Nocken, die eine schraubenförmige Neigung aufweisen. Diese Nocken gelangen durch Verdrehen dieses Drehelementes in entsprechende schlitzförmige Ausnehmungen der beiden Schenkel der U-förmigen Ausnehmung. Durch Verdrehen dieses Drehelementes wird die Stange klemmend gehalten. Um ein Lösen dieses Drehelementes vermeiden zu können, ist die Spannfläche, die gegen die Stange gerichtet ist, mit querverlaufenden Ausnehmungen versehen, die bei Erreichen der Klemmposition in die Oberfläche der Stange einklinken sollen.

Diese Einrichtung ist einfach zu handhaben, es ist aber schwierig, mit den vorgegebenen Positionen der Ausnehmungen, die in die Stange einklinken sollen, die richtige Spannkraft erhalten zu können, damit die Stange in optimaler Weise in der Pedikelschraube festgehalten wird. Zusätzlich besteht die Gefahr, dass die beiden Schenkel durch die Nocken des Drehelementes elastisch auseinandergedrückt werden, was die gewünschte Spannkraft beeinträchtigen kann.

Zudem ist aus der EP 1 234 547 A1 eine Knochenschraube bekannt, bei welcher die Verschlusseinrichtung aus einem verdrehbaren Element mit Bajonettverschluss und einer in dieses einschraubbaren Spannschraube gebildet ist. Diese Verschlusseinrichtung ist nicht einfach auf die Knochenschraube aufsetzbar, zudem können durch die Einwirkung der Spannschaube auf den Stab an diesem Beschädigungen auftreten.

Die WO-A-95/01132 und die WO-A-2005/102195 offenbaren jeweils eine Pedikelschraube mit einem Einschraubteil, einem Kopfteil mit einer U-förmigen Ausnehmung und zwei Schenkeln, einem Verriegelungselement, das einen Kragen und gegeneinander gerichtete Nocken aufweist, außenseitig an den Schenkeln verlaufende Nuten, wobei das Verriegelungselement auf die Schenkel aufsetzbar ist und die Nocken in die Nuten gelangen; eine Spannschraube ist in das Verriegelungselement eingeschraubt, und an der Spannschraube ist ein Spannelement angeordnet.

Die Aufgabe der vorliegenden Erfindung besteht somit darin, eine Pedikelschraube mit einer Verschlusseinrichtung zur Befestigung eines Stabes zur Stabilisierung der Wirbelsäule zu schaffen, welche einfach zu har dhaben ist, und mit welcher die gewünschte und optimale Spannkraft bei jede Art von verwendeten Stangen erreicht werden kann.

Erfindungsgemäss erfolgt die Lösung dieser Aufgabe dadurch, dass an der Spannschraube ein Spannelement angeordnet ist, welches verdrehbar mit der Spannschraube verbunden ist, dass das Spannelement mit Fihrungen ausgestattet ist, welche aus an dem Spannelement angebrachten Führungsrippen bestehen, welche mit an den Schenkeln angebrachten Führungsflächen zusammenwirken, mittels welchen das Spannelement geführt zwischen die beiden Schenkel des Kopfteils einsatzbar ist, und dass das zwischen die Schenkel eingeführte Spannelement durch die Spannschraube gegen den in die Ausnehmung eingesetzten Stab spannbar ist.

Diese Verschlusseinrichtung lässt sich sehr einfach handhaben, das Spannelement wird zwischen die Schenkel, welche die U-förmige Ausnehmung der Pedikelschraube für die Aufnahme der Stange bilden, nach dem Einsetzen der Stange eingefahren, das Verriegelungselement wird durch Verdrehen in die Verriegelungsposition gebracht, die Spannschraube kann angezogen werden, so dass das Spinnelement mit der gewünschten Kraft gegen die zu fixierende Stange gepresst wird. Durch das Anpressen des Spannelementes gegen die Stange wird erreicht, dass die Nocken des Verriegelungselementes rrit der entsprechenden Gegenkraft gegen die entsprechende Fläche der jeweiligen Nut gepresst werden, so dass auch das Verriegelungselement durch Reibschluss entsprechend gesichert ist und ein Zurückdrehen des Verriegelungselementes verunmöglicht wird.

Zudem ist das Spannelement mit Führungen ausgestattet, welche Führungen aus an dem Spannelement angebrachten Führungsrippen bestehen, welche mit an den Schenkeln angebrachten Führrungsflächen zusammenwirken, mittels welchen das Spannelement geführt auf die beiden Schenkel des Kopfteils aufsetzbar ist, was zur Vereinfachung der Handhabung beiträgt.

Ferner sind die Spannschraube und das Spannelement verdrehbar mit einander verbunden sind. Dadurch kann erreicht werden, dass eir e vormontierte Verschlusseinrichtung mit dem Verriegelungselement, ir dieses eingeschraubte Spannschraube und damit verbundenem Spannelement als Einheit auf die Pedikelschraube aufgesetzt werden kann.

In vorteilhafter Weise ist die Verriegelungsposition des bügelförmigen Verriegelungselementes im Kopfteil durch Anschläge festgelegt. Dadurch ist gewährleistet, dass die korrekte Verriegelungsposition des bügelförmigen Verriegelungselementes erreicht wird.

Die verdrehbare Verbindung zwischen Spannschraube und Spannelement wird in vorteilhafter Weise dadurch erreicht, dass die Spannschraube einen Gewindeteil aufweist, welcher mit einem Bolzenstück verlängert ist, und zwischen Gewindeteil und Bolzenstück eine Abstützfläche gebildet ist. Zur Aufnahme des Bolzenstücks ist das Spannelement mit einer zentralen Bohrung ausgestattet, der dem Gewindeteil abgewandte Endbereich des Bolzenstücks ist mit einer Verdickung versehen, die in einfacher und vorteilhafter Weise durch Aufkrempen dieses Endbereichs erreicht werden kann.

In vorteilhafter Weise stützt sich die Abstützfläche auf einer entsprechend am Spannelement angebrachten Fläche ab, wodurch e ne optimale Übertragung der Spannkräfte erreichbar ist.

Eine weitere vorteilhafte Ausgestaltung der Erfindung besteht darin, dass der Stab aus einem elastischen Material gebildet ist, dessen zyl ndrische Oberfläche mit Rippen und Rillen versehen ist, und dass die die inneren Oberflächen der Schenkel verbindende Grundfläche und die gegen den Stab gerichtete Fläche des bügelförmigen Verriegelungselementes mit entsprechenden Rippen und Rillen versehen ist. Dadurch wird eine optimale formschlüssige Verbindung zwischen Stab und Pedikelschraube erreicht, was insbesondere vorteilhaft ist, wenn der Stab aus einem elastischen Material gebildet ist, beispielsweise aus einem biokompatiblen Kunststoff, insbesondere auf der Basis von Polyurethan.

Eine Ausführungsform der Erfindung wird nachfolgend anhand der beiliegenden Zeichnung beispielhaft näher erläutert.

Es zeigt
Fig. 1 in räumlicher Darstellung eine Pedikelschraube mit einer Verschlusseinrichtung, bestehend aus einem Spannelement, einem Verriegelungselement und der Spannschraube, in auseinandergezogenem Zustand;
Fig. 2 im Schnitt eine Darstellung der Verschlusseinrichtung gemäss Fig. 1 im verbundenen Zustand;
Fig. 3 in räumlicher Darstellung die Pedikelschraube mit zum Aufsetzen bereiter Verschlusseinrichtung;
Fig. 4 in räumlicher Darstellung die Pedikelschraube mit aufgesetzter Verschlusseinrichtung, bereit zum Verriegeln;
Fig. 5 in räumlicher Darstellung die Pedikelschraube mit aufgesetzter und verriegelter Verschlusseinrichtung;
Fig. 6 eine Schnittdarstellung durch die Pedikelschraube mit aufgesetzter und verriegelter Verschlusseinrichtung; und
Fig. 7 in räumlicher Darstellung die Pedikelschraube, den in die Pedikelschraube einzusetzenden Stab und das Spannelement.

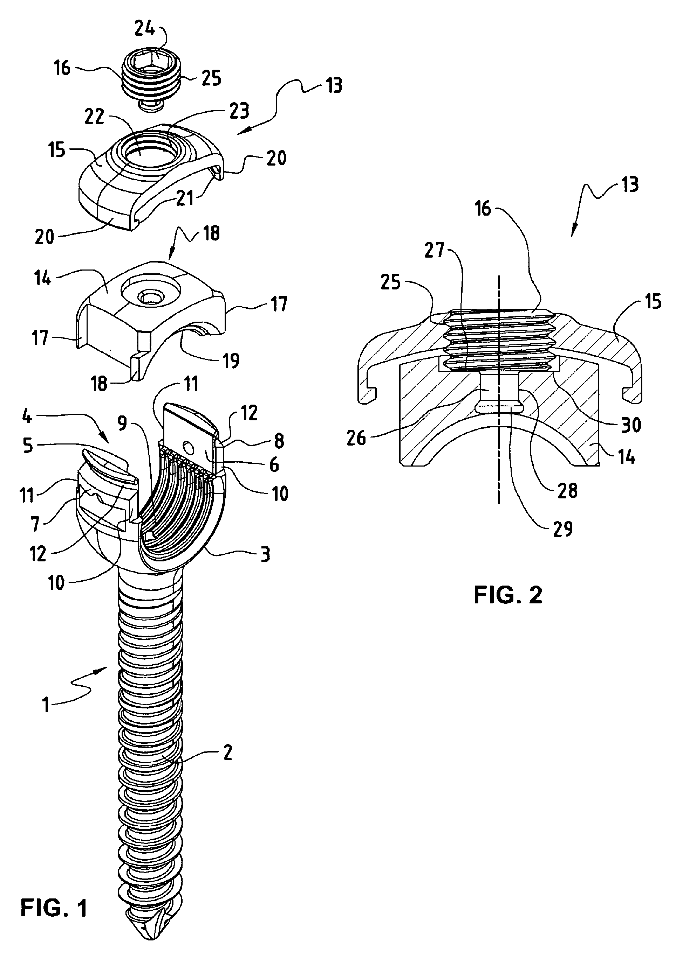

Wie aus Fig. 1 ersichtlich ist, besteht die Pedikelschraube 1 aus einem mit einem Gewinde versehenen Einschraubteil 2, mit welchem die Pedikelschraube 1 in bekannter Weise in einen Wirbelkörper einer Wirbelsäule eines Patienten eingeschraubt werden kann. An diesem Einschraubteil 2 ist ein Kopfteil 3 angebracht, welcher mit einer U-förmigen Ausnehmung 4 versehen ist. Diese U-förmige Ausnehmung 4 wird durch die inneren Oberflächen 5, 6 zweier Schenkel 7, 8 gebildet, welche am Einschraubteil 2 befestigt sind. Die beiden inneren Oberflächen 5 und 6 dieser beiden Schenkel werden durch eine Grundfläche 9 miteinander verbunden, welche Grundfläche 9 der Form des in diese U-förmige Ausnehmung 4 einzusetzenden Stabes angepasst ist, wie später noch gesehen wird.

Die beiden Seitenbereiche der jeweiligen Schenkel 7 und 8 sind jeweils mit einer Führungsfläche 10, 11 ausgestattet. Im oberen Bereich ist aussenseitig an den beiden Schenkeln 7 und 8 jeweils eine quer zu diesen verlaufende Nut 12 angebracht. Der Nutgrund und die die Nut 12 begrenzenden Flächen sind kreisbogenförmig ausgebildet.

Die U-förmige Ausnehmung 4 des Kopfteils 3 der Pedikelschraube 1 kann durch eine Verschlusseinrichtung 13 verschlossen werden. Diese Verschlusseinrichtung 13 setzt sich zusammen aus einem Spannelement 14, einem Verriegelungselement 15 und einer Spannschraube 16. Das Spannelement 14 lässt sich zwischen die beiden Schenkel 7 und 8 einsetzen. Hierzu ist dieses Spannelement 14 mit jeweils zwei einander gegenüberliegenden Führungsrippen 17 und 18 ausgestattet, welche mit den Führungsflächen 10 und 11 der beiden Schenkel 7 und 8 die Führung bilden. Die der Grundfläche 9 zugewandte Oberfläche 19 des Spannelementes 14 weist ebenfalls eine der Form des Stabes angepasste Form auf.

Das Verriegelungselement 15 weist die Form eines Bügels auf, und ist an den beiden einander gegenüberliegenden Endbereichen jeweils mit einem Kragen 20 ausgestattet. An jedem dieser Kragen 20 ist ein Nocken 21 angebracht, die gegeneinander gerichtet sind. Zusätzlich ist das Verriegelungselement 15 mit einer Bohrung 22 ausgestattet, welche mit einem Gewinde 23 versehen ist. In das Gewinde 23 dieser Bohrung 22 lässt sich die Spannschraube 16 einschrauben, welche mit einem Innensechskant 24 ausgestattet ist. Mit dieser Verschlusseinrichtung 13 lässt sich die U-förmige Ausnehmung 4 im Kopfteil 3 der Pedikelschraube 1 verschliessen, der in diese U-förmige Ausnehmung eingelegte Stab wird dadurch festgehalten, wie später noch gesehen wird.

Wie aus Fig. 2 ersichtlich ist, wird die Verschlusseinrichtung 13 zu einer Einheit zusammengesetzt. Die Spannschraube 16 ist mit einem Gewindeteil 25 versehen, an welchem ein Bolzenstück 26 angesetzt ist. Zwischen dem Gewindeteil 25 und dem Bolzenstück 26 ist eine Abstützfläche 27 gebildet. Das Bolzenstück 26 ragt in eine zentrale Bohrung 28, welche im Spannelement 14 angebracht ist. Der dem Gewindeteil 25 abgewandte Endbereich des Bolzenstücks 26 ist mit einer Verdickung 29 ausgestattet, welche beispielsweise durch Aufkrempen des Endbereichs dieses Bolzenstücks 26 erreichbar ist, welcher Aufkrempvorgang nach dem Einsetzen der Spannschraube 16 in das Spannelement 14 ausgeführt werden kann. Die Spannschraube 16 und das Spannelement 14 sind somit um das Bolzenstück 26 verdrehbar miteinander verbunden. Die Abstützfläche 27 der Spannschraube 16 stützt sich auf einer entsprechend am Spannelement 14 angebrachten Fläche 30 ab. Die Spannschraube 16 mit dem mit diesem verbundenen Spannelement lässt sich dann in das Verriegelungselement 15 einschrauben, wodurch die Verschlusseinrichtung 13 eine Einheit bildet.

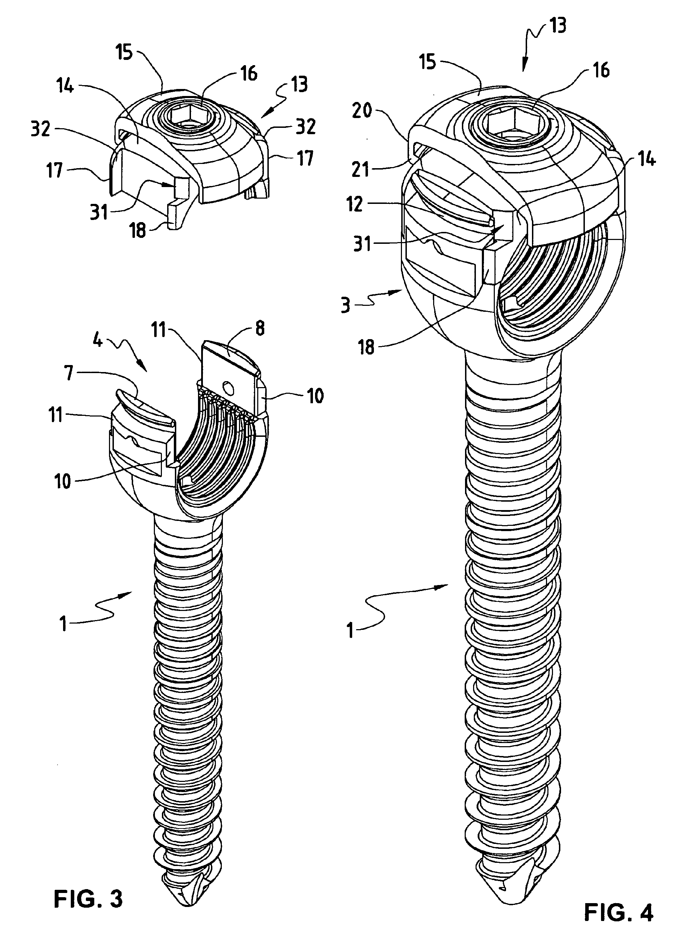

Diese zu einer Einheit verbundenen Verschlusseinrichtung 13 lässt sich dann auf den Kopfteil 3 der Pedikelschraube 1 aufsetzen. Wie aus Fig. 3 ersichtlich ist, wird hierzu das Verriegelungselement 15 bezüglich des Spannelementes 14 quer ausgerichtet. Die Verschlusseinrichtung 13 kann dann in die U-förmige Ausnehmung 4 der Pedikelschraube eingefahren werden. Hierzu werden die Führungsrippen 17 und 18 des Spannelementes 14 durch die Führungsflächen 10 und 11 der beiden Schenkel 7 und 8 geführt. Wie aus Fig. 3 ersichtlich ist, ist die jeweilige Führungsrippe 18 des Spannelementes 14 mit einer Aussparung 31 versehen, wodurch erreicht wird, dass das Verriegelungselement 15 bezüglich des Spannelementes 14 um die Achse der Spannschraube 16 über die jeweilige Führungsrippe 18 verdreht werden kann. Die jeweiligen Führungsrippen 17 dienen beim Verdrehen bzw. Verriegeln des Verriegelungselementes 15 als Anschlag 32, die Verriegelungsposition ist erreicht, wenn das Verriegelungselement 15 gegen diese Anschläge 32 stösst.

In Fig. 4 ist die Situation dargestellt, bei welcher die Verschlusseinrichtung 13 auf den Kopfteil 3 der Pedikelschraube 1 aufgesetzt aber noch nicht verriegelt ist. Zum Verriegeln wird nun das Verriegelungselement 15 im Uhrzeigersinn gedreht, die am Kragen 20 angebrachten Nocken 21 gelangen in die Nut 12, zum Einfahren dieser Nocken 21 in die Nut 12 durchfährt der jeweilige Nocken 21 die Aussparung 31, die an der jeweiligen Führungsrippe 18 des Spannelementes 14 vorgesehen ist.

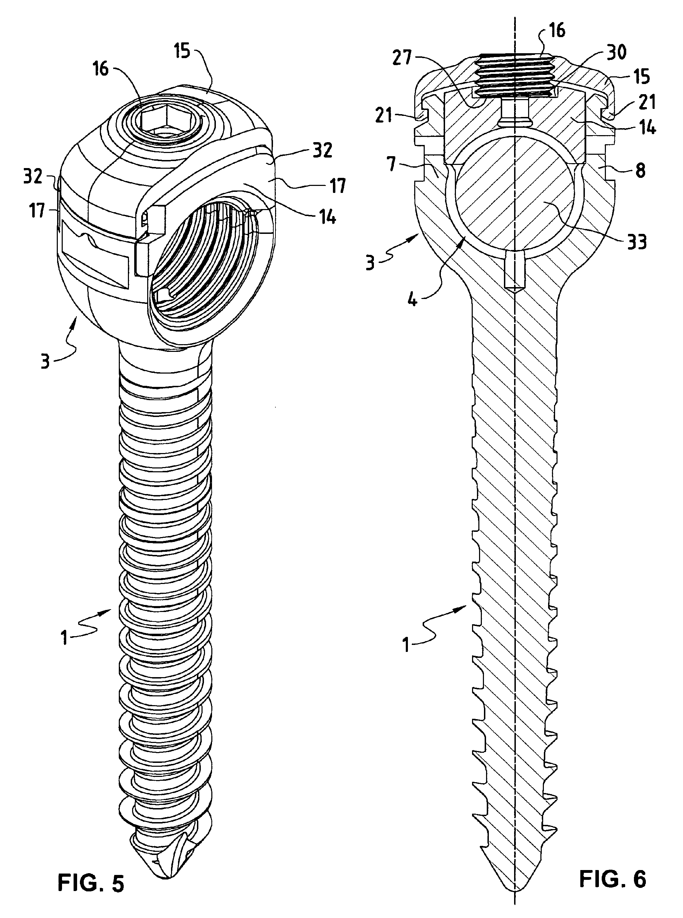

In Fig. 5 ist dargestellt, wenn das Verriegelungselement 15 die Verriegelungsposition erreicht hat. Hierbei stösst das Verriegelungselement 15 jeweils am Anschlag 32 an, der an der Führungsrippe 17 des Spannelements 14 angebracht ist.

Selbstverständlich wird, wenn eine Pedikelschraube in den Wirbelkörper einer Wirbelsäule eingesetzt ist, vor dem Aufsetzen der Verschlusseinrichtung 13 der Stab 33 eingesetzt, wie das in der Schnittdarstellung gemäss Fig. 6 ersichtlich ist. In der Schnittdarstellung gemäss Fig. 6 befindet sich die Verschlusseinrichtung 13 im auf die Pedikelschraube 1 aufgesetzten Zustand, das Verriegelungselement 15 befindet sich in der Verriegelungsposition, wie dies in Fig. 5 dargestellt ist. In dieser Situation kann nun die Spannschraube 16 angezogen werden. Beim Anziehen dieser Spannschraube 16 drückt die Abstützfläche 27 der Spannschraube 16 auf die Fläche 30 des Spannelementes 14. Hierbei wird das Spannelement 14 gegen den Stab 33 gepresst, die auftretende Gegenkraft wird über das Verriegelungselement 15 und über die Nocken 21 auf den Kopfteil 3 der Pedikelschraube 1 übertragen. Der Stab 33 wird somit in der U-förmigen Ausnehmung 4 und dem Spannelement 14 verspannt, gleichzeitig wird erreicht, dass das Verriegelungselement 15 durch die entsprechende auftretende Gegenkraft gegen Verdrehen gesichert ist. Dadurch wird in optimaler Weise der Stab 33 im Kopfteil 3 der Pedikelschraube 1 fixiert.

Indem das Verriegelungselement 15 die beiden Schenkel 7 und 8 der U-förmigen Ausnehmung 4 von aussen umschliesst, werden diese beiden Schenkel 7 und 8 auch im verspannten Zustand in der richtigen Position gehalten, das heisst ein Aufspreizen der beiden Schenkel 7 und 8 wird verunmöglicht. Dadurch lässt sich der Kopfteil 3 dieser Pedikelschraube 1 sehr kompakt bauen. Insbesondere kann die Bauhöhe gering gehalten werden.

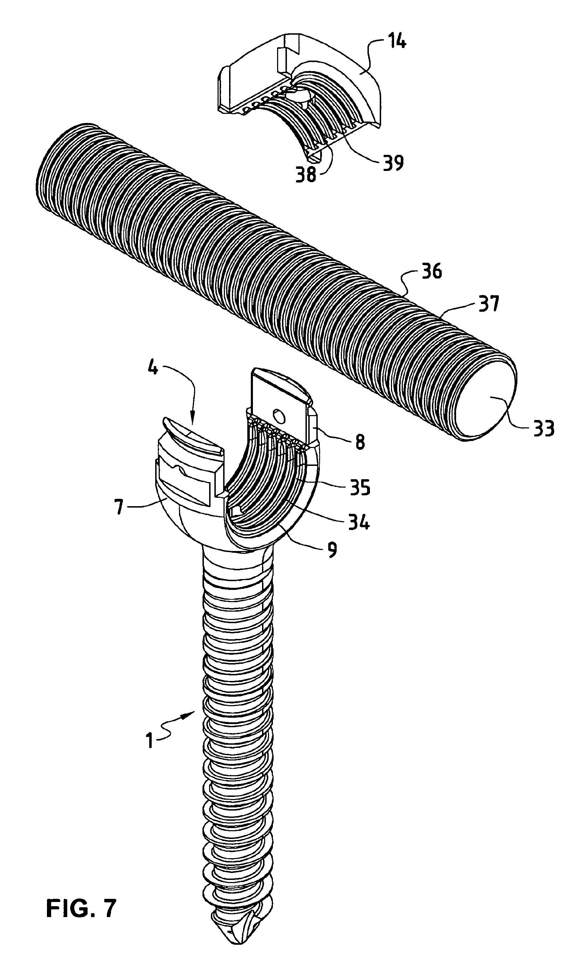

Wie in Fig. 7 dargestellt ist, ist bei dieser Pedikelschraube 1 die die inneren Oberflächen der Schenkel 7 und 8 verbindende Grundfläche 9 mit quer zum Stab 33 verlaufenden Rippen 34 und Rillen 35 versehen. Entsprechende Rippen 36 und Rillen 37 weist der Stab 33 auf. Auch das Spannelement 14 ist mit entsprechenden Rippen 38 und Rillen 39 versehen. Im in die U-förmige Ausnehmung 4 eingesetzten Zustand des Stabes 33 greifen die Rippen 34 der Pedikelschraube in die Rillen 37 des Stabes ein, während die Rippen 36 des Stabes in die entsprechenden Rillen 35 der Pedikelschraube eingreifen. Entsprechend greifen die Rippen 38 des Spannelementes in die Rillen 37 des Stabes ein, während die Rippen 36 des Stabes 33 in die Rillen 39 des Spannelementes 14 eingreifen. Dadurch erhält man eine formschlüssige Verbindung zwischen Pedikelschraube 1, Stab 33 und Spannelement 14 bzw. der Verschlusseinrichtung 13, dies ist insbesondere dann vorteilhaft, wenn der Stab aus einem elastischen Material gebildet ist, beispielsweise aus einem biokompatiblen Kunststoff auf Basis von Polyurethan.

Derartige Pedikelschrauben sowie die Verschlusseinrichtung werden üblicherweise aus einer Titanlegierung hergestellt. Selbstverständlich wären aber auch andere geeignete Materialien denkbar.

Im in der Zeichnung dargestellten Ausführungsbeispiel sind Pedikelschraube 1 und Spannelement 14 der Verschlusseinrichtung 13 sowie der Stab 33 mit entsprechenden Rippen und Rillen ausgestattet. Selbstverständlich könnten die entsprechenden Oberflächen glatt sein, insbesondere wenn ein relativ starrer Stab, beispielsweise aus einer Titanlegierung, verwendet wird. Beim Befestigen dieses mit einer glatten Oberfläche versehenen Stabes in der Pedikelschraube wird eine reine Reibschlussverbindung erhalten, welche aber durch entsprechendes Festziehen der Spannschraube ebenfalls eine sichere Fixierung ergibt.

Mit dieser Ausgestaltung wird eine Pedikelschraube mit Verschlusseinrichtung erhalten, mit welcher sowohl elastische Stäbe wie auch starre Stäbe in optimaler Weise in der jeweiligen Pedikelschraube fixiert werden können, durch die dargestellte Ausgestaltung erreicht man eine sehr kompakte Bauweise, zudem ist das Einsetzen des Stabes in die Pedikelschraube, das Anbringen der Verschlusseinrichtung, das Verriegeln und das Verspannen sehr einfach möglich und benötigt lediglich sehr einfache Werkzeuge.

## Patentansprüche

1. Pedikelschraube mit einer Verschlusseinrichtung (13) zur Befestigung eines Stabes (33) zur Stabilisierung der Wirbelsäule, umfassend einen Einschraubteil (2), einen am Einschraubteil (2) angebrachten Kopfteil (3), eine im Kopfteil (3) angeordnete U-förmige Ausnehmung (4), die durch die inneren Oberflächen (5, 6) zweier Schenkel (7, 8) und einer die beiden inneren Oberflächen (5, 6) verbindenden Grundfläche (9) gebildet ist, in welche Ausnehmung (4) der Stab (33) einsetzbar und durch die Verschlusseinrichtung (13) gehalten ist, welche Verschlusseinrichtung (13) ein bügelförmiges Verriegelungselement (15) umfasst, welches an den beiden einander gegenüberliegenden Endbereichen jeweils einen Kragen (20) aufweist, an welchen jeweils gegeneinander gerichtete Nocken (11) angebracht sind, und aussenseitig an den beiden Schenkeln (7, 8) jeweils eine quer zu diesen vorlaufende Nut (12) angebracht ist, derart dass das bügelförmige Verriegelungselement (15) auf die Schenkel (7, 8) aufsetzbar und verdrehbar ist und die Nocken (21) zum Erreichen der Verriegelungsposition jeweils in die entsprechende (12) Nut gelangen, und in das bügelförmige Verriegelungselement (15) eine Spannschraube (16) eingeschraubt ist, wobei an der Spannschraube (16) ein Spanr element (14) angeordnet ist, welches verdrehbar mit der Spannschraube (16) verbunden ist, das Spannelement (14) mit Führungen ausgestattet ist, welche aus an dem Spannelement (14) angebrachten Führungsrippen (17, 18) bestehen, welche mit an den Schenkeln (7, 8) angebrachten Führungsflächen (10, 11) zusammenwirken, mittels welchen das Spannelement (14) geführt zwischen die beiden Schenkel (7, 8) des Kopfteils (3) einsetzbar ist, und wobei das zwischen die Schenkel (7, 8) eingeführte Spannelement (14) durch die Spannschraube (16) gegen den in die Ausnehmung (4) eingesetzten Stab (33) spannbar ist.

2. Pedikelschraube mit einer Verschlusseinrichtung nach Anspruch 1, wobei die Verriegelungsposition des bügelförmigen Verriegelungselementes (13) im Kopfteil (3) durch Anschläge (32) festgelegt ist.

3. Pedikelschraube mit einer Verschlusseinrichtung nach Anspruch 1 oder 2, wobei die Spannschraube (16) einen Gewindeteil (25) aufweist, welcher mit einem Bolzenstück (26) verlängert ist, und dass zwischen Gewindeteil (25) und Bolzenstück (26) eine Abstützfläche (27) gebildet ist.

4. Pedikelschraube mit einer Verschlusseinrichtung nach Anspruch 3, wobei das Spannelement (14) mit einer zentralen Bohrung (28) ausgestattet ist, in welche das Bolzenstück (26) der Spannschraube (16) hineinragt, und dass der dem Gewindeteil (25) abgewandte Endbereich des Bolzenstücks (26) mit einer Verdickung 29) versehen ist.

5. Pedikelschraube mit einer Verschlusseinrichtung nach Anspruch 3 oder 4, wobei sich die Abstützfläche (27) auf einer entsprechend am Spannelement (14) angebrachten Fläche (30) abstiitzt.

6. Pedikelschraube mit einer Verschlusseinrichtung nach einem der Ansprüche 1 bis 5, wobei der Stab (33) aus einem elastischen Material gebildet ist, dessen zylindrische Oberfläche mit Rippen (36) und Rillen (37) versehen ist, und dass die die inneren Oberflächen (5, 6) der Schenkel (7, 8) verbindende Grundfläche (9) und die gegen die Stange (33) gerichtete Fläche des bügelförmigen Verriegelungselements (15, mit entsprechenden Rippen (36; 38) und Rillen (37; 39) versehen ist.

7. Pedikelschraube mit einer Verschlusseinrichtung nach Anspruch 8, wobei der elastische Stab (33) aus einem biokompatiblen Kunststoff, insbesondere auf Basis von Polyurethan, gebildet ist.

## Claims

1. Pedicle screw with a closure device (13) for securing a rod (33) for stabilization of the vertebral column, comprising a screw-in part (2), a head part (3) provided on the screw-in part (2), a U-shaped recess (4) disposed in the head part (3), which recess is formed by the inner surfaces (5, 6) of two arms (7, 8) and a bottom surface (9) connecting the two inner surfaces (5, 6), in which recess (4) the rod (33) is insertable and is held by the closure device (13), which closure device (13) comprises a yoke-shaped locking element (15) which has a collar (20) on each of the two end regions opposite each other, on each of which collars projections (21) are provided, directed toward each other, and a groove (12) is provided outside on each of the two arms (7, 8) running transversely to these arms such that the yoke-shaped locking element (15) is placeable on the arms (7, 8) and is rotatable, and, to reach the locking position, the projections (21) each end up in the corresponding groove (12), and a tension screw (16) is screwable into the yoke-shaped locking element (15), a tension element (14) being disposed on the tension screw (16), which element is connected to the tension screw (16) in a rotatable way, the tension element (14) being provided with guides consisting of guide ribs (17, 18) disposed on the tension element (14), which ribs co-operate with guide surfaces (10, 11) disposed on the arms (7, 8), by means of which the tension element (14) is insertable in a guided way between the two arms (7, 8) of the head part (3) and the tension element (14) inserted between the arms (7, 8) being able to be tensioned by the tension screw (16) against the rod (33) inserted in the recess (4).

2. Pedicle screw with a closure device according to claim 1, the locking position of the yoke-shaped locking element (13) in the head part (3) is determined by stops (32).

3. Pedicle screw with a closure device according to claim 1 or 2, the tension screw having a threaded part (25) that is extended by a bolt piece (26), and a support surface (27) being formed between threaded part (25) and bolt piece (26).

4. Pedicle screw with a closure device according to claim 3, the tension element being provided with a central bore (28), into which the bolt piece (26) of the tension screw (16) protrudes, and the end region of the bolt piece (26) remote from the threaded part (25) being provided with a swelling (29).

5. Pedicle screw with a closure device according to claim 3 or 4, the support surface (27) being supported on a surface (30) correspondingly provided on the tension element (14).

6. Pedicle screw with a closure device according to one of the claims 1 to 5, the rod (33) being made of an elastic material, the cylindrical surface of which is provided with ridges (36) and grooves (37), and the bottom surface (9) connecting the inner surfaces (5, 6) of the arms (7, 8) and the surface of the yoke-shaped locking element (15) turned toward the rod (33) being provided with corresponding ridges (36; 38) and grooves (37; 39).

7. Pedicle screw with a closure device according to claim 8 <sic. 6>, the elastic rod (33) being made of a biocompatible plastic, in particular a polyurethane-based material.

## Revendications

1. Vis pédiculaire avec un dispositif de fermeture (13) pour la fixation d'une barre (33) pour la stabilisation de la colonne vertébrale, comprenant une partie de vissage (2), une partie de tête (3) attachée à la partie de vissage (2), une cavité (4) en forme de U disposée sur la partie de tête (3) qui est formée par les surfaces internes (5, 6) de deux manches (7, 8) et une surface de base (9) connectant les deux surfaces internes (5, 6), dans laquelle cavité (4) la barre (33) est insérable et est tenue par le dispositif de fermeture (13), lequel dispositif de fermeture (13) comprend un élément de verrouillage (15) en forme de poignée, qui a un col (20) sur chacune des deux extrémités en face l'une de l'autre, sur chacun desdits cols des cames (21) dirigées l'une contre l'autre sont prévues, et à l'extérieur des deux manches (7, 8) une rainure (12) traversant ces manches est prévue, de manière que l'élément de verrouillage (15) en forme de poignée soit positionnable sur les manches (7, 8) et rotatif et que, pour atteindre la position de verrouillage, les cames (21) se terminent chacune dans la rainure (12) correspondante, et qu'une vis de tension (16) est vissée dans l'élément de verrouillage (15) en forme de poignée, un élément de tension (14) étant disposé sur la vis de tension (16), lequel élément est connecté à la vis de tension (16) d'une manière rotative, l'élément de tension (14) étant prévu avec des guides, qui consistent en des nervures de guidage (17, 18) disposées sur l'élément de tension (14), lesquelles nervures sont concomitantes avec des surfaces de guidage (10, 11) disposées sur les manches (7, 8), au moyen desquelles l'élément de tension (14) guidé entre les deux manches (7, 8) de la tête (3) est insérable, et l'élément de tension (14) inséré entre les manches (7, 8) pouvant être tendu par la vis de tension (16) contre la barre (33) insérée dans la cavité (4).

2. Vis pédiculaire avec un dispositif de fermeture (13) selon la revendication 1, la position de verrouillage de l'élément de verrouillage en forme de poignée (13) dans la partie de tête (3) étant déterminée par des butées (32).

3. Vis pédiculaire avec un dispositif de fermeture (13) selon la revendication 1 ou 2, la vis de tension (16) comprenant une partie de filetage (25), qui est étendue avec une pièce de boulon (26) et une surface d'étaiement (27) étant formée entre la partie de filetage (25) et la pièce de boulon (26).

4. Vis pédiculaire avec un dispositif de fermeture (13) selon la revendication 3, l'élément de tension (14) étant prévu avec un forage central (28), dans lequel la pièce de boulon (26) de la vis de tension (16) dépasse et l'extrémité de la pièce de boulon (26) appliquée sur la partie de filetage (25) étant munie d'une surépaisseur (29).

5. Vis pédiculaire avec un dispositif de fermeture (13) selon la revendication 3 ou 4, la surface d'étaiement (27) s'appuyant sur une surface correspondante (30) disposée sur l'élément de tension (14).

6. Vis pédiculaire avec un dispositif de fermeture (13) selon l'une quelconque des revendications 1 à 5, la barre (33) étant formée d'un matériau élastique dont la surface cylindrique est munie de nervures (36) et de rainures (37), et la surface de base connectant les surfaces internes (5, 6) des manches (7, 8) et la surface de l'élément de verrouillage en forme de poignée (15) dirigée vers la barre (33) étant prévues avec les nervures (36, 38) et rainures (37, 39) correspondantes.

7. Vis pédiculaire avec un dispositif de fermeture (13) selon la revendication 8, la barre élastique (33) étant formée d'un plastique biocompatible, en particulier à base de polyuréthane.
